## Europäisches Patentamt

## European Patent Office

## Office européen des brevets

(11) Veröffentlichungsnummer: **0 054 689**
**B1**

(12) **EUROPÄISCHE PATENTSCHRIFT**

(45) Veröffentlichungstag der Patentschrift:
29.08.84

(21) Anmeldenummer: 81108946.5

(22) Anmeldetag: 27.10.81

(51) Int. Cl.³: **G 01 N 33/52**, G 01 N 33/82,
C 07 D 257/04, C 07 D 417/04 //
(C07D417/04, 257/04, 277/38)

(54) Stabilisierte Zubereitung von Tetrazoliumsalzen.

(30) Priorität: 23.12.80 DE 3048662

(43) Veröffentlichungstag der Anmeldung:
30.06.82 Patentblatt 82/26

(45) Bekanntmachung des Hinweises auf die Patenterteilung:
29.08.84 Patentblatt 84/35

(84) Benannte Vertragsstaaten:
AT BE CH DE FR GB IT LI LU NL SE

(56) Entgegenhaltungen:
EP - A - 0 007 058
DE - A - 2 147 466
DE - A - 2 459 087
DE - A - 2 537 499
DE - A - 2 803 955
FR - A - 2 314 497
US - A - 3 783 105
US - A - 4 026 767

FETTE, SEIFEN, ANSTRICHMITTEL, Band 75, Heft 6,
Juni 1973, V.A. RÖDER: "Enzymatische Analyse -
Grundlagen, Fortschritte, Grenzen", Seiten 395-397

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Geisler, Edda, Dipl.-Ing. grad. Chem., Feldberg
Strasse 58, D-6800 Mannheim 23 (DE)
Erfinder: Feuerstein, Helmut, Neue Schulstrasse 55,
D-6804 Mannheim-Ilvesheim (DE)
Erfinder: Lange, Hans-Rudolf, Danziger Strasse 3,
D-6840 Lampertheim (DE)

## Beschreibung

Gegenstand der Erfindung ist eine stabilisierte Zubereitung von Tetrazoliumsalzen für analytische Zwecke, ein Verfahren zum Nachweis von reduzierenden Stoffen mittels Tetrazoliumsalzen und eine Verwendung dieser Zubereitung zum Nachweis von NADH,NADH-bildenden Systemen oder Ascorbinsäure.

Tetrazoliumsalze sind in der analytischen Chemie zum Nachweis reduzierender Stoffe, insbesondere von NADH seit langem bekannt. Die Wasserstoffübertragung wird außer durch Enzyme wie Diaphorase, auch durch 5-Methylphenazinium-methylsulfat (PMS) oder ähnliche Substanzen katalysiert, wobei sich tiefgefärbte Formazane bilden, die einen sehr empfindlichen Nachweis der reduzierenden Substanzen im sichtbaren Licht erlauben. Es sind deshalb entsprechende Verfahren entwickelt worden, eine Reihe von in der analytischen Chemie wichtigen Substanzen über das als Zwischenprodukt gebildete NADH auf diese Art nachzuweisen. Nachteilig ist bei dieser Reaktion, daß sich die Tetrazoliumsalze insbesondere in Lösung relativ leicht zersetzen und farbige Zersetzungsprodukte bilden. Selbst bei Lagerung bei tiefen Temperaturen und Ausschluß von Licht ist deshalb die Stabilität solcher Produkte begrenzt (H. U. Bergmeyer, Grundlagen der enzymatischen Analyse, Verlag Chemie, Weinheim 1977, Seite 91—95 mit weiteren Nachweisen).

Es stellt sich deshalb die Aufgabe, Stabilisierungsmittel zu finden, die die Empfindlichkeit der Tetrazoliumsalze gegen höhere Temperaturen und Licht vermindern oder beseitigen, ohne dadurch die Empfindlichkeit der Nachweisreaktion zu beeinflussen.

Erfindungsgemäß werden stabilisierte Zubereitungen von Tetrazoliumsalzen für analytische Zwecke vorgeschlagen, die 1—10 Mol, vorzugsweise 1—2 Mol Borsäure oder einer organischen, in polaren Lösungsmitteln löslichen Hydroxylpolycarbonsäure pro Mol Tetrazoliumsalz enthalten. Weitere Merkmale und ein Verfahren zum Nachweis von reduzierenden Stoffen mittels Tetrazoliumsalzen sowie die Verwendung der erfindungsgemäßen Zubereitungen sind in den Patentansprüchen näher gekennzeichnet.

Die üblicherweise verwendeten Tetrazoliumsalze wie

3-(4',5'-dimethyl-thiazolyl-2-)-2,4-diphenyl-tetrazoliumbromid (MTT)
2-(p-Iodophenyl)-3-(p-nitrophenyl)-5-phenyl-tetrazoliumchlorid (INT)
2,2',5,5'-Tetra-(p-nitrophenyl)-3,3'-(3-dimethoxy-4-diphenylen)-ditetrazoliumchlorid (TNBT)
2,2'-Di(p-nitrophenyl)-5,5'-diphenyl-3,3-(3,3'-dimethoxy-4,4'-diphenylen)-
ditetrazoliumchlorid (NBT)
2,2'-p-Diphenylen-3,3',5,5'-tetraphenyl-ditetrazoliumchlorid (Neotetrazoliumchlorid) (NT)
2,3,5-Triphenyl-tetrazoliumchlorid (TT)

werden üblicherweise als Chloride oder Bromide im Handel angeboten und wurden als solche in bisherige Teste eingesetzt. Eine Vermutung, daß diese Salze in Lösung teilweise hydrolysieren und die Instabilität in irgendeiner Weise damit zusammenhängt, konnte nicht bestätigt werden, da ein Zusatz von üblichen Säuren die Instabilität nicht beseitigt. Übliche starke Säuren wie Toluolsulfosäure, Oxalsäure oder Malonsäure erwiesen sich als wirkungslos.

Überraschenderweise wurde nun gefunden, daß teilweise weniger saure, aber komplexbildende Säuren, nämlich Borsäure oder organische Hydroxypolycarbonsäuren, beispielsweise Zitronensäure oder Äpfelsäure, einen ganz erheblichen stabilisierenden Effekt, sowohl gegen Belastung der Zubereitung durch Temperatur, als auch gegen Belichtung, besitzen. Lösungen einer solchen Zubereitung können beispielsweise mehrere Tage bei Raumtemperatur und Tageslicht aufbewahrt werden ohne sich merklich zu verändern, d. h. ihre Brauchbarkeit für analytische Zwecke zu verlieren. Handelsübliche Zubereitungsformen wie Lyophilisate oder Imprägnierungen auf saugfähigen Trägern (Reagenzstreifen), können nach der erfindungsgemäßen Stabilisierung nach bisherigen Befunden mindestens ein Jahr lang bei Raumtemperatur ohne merkliche Zersetzung aufbewahrt werden.

Da die erfindungsgemäß eingesetzten Stabilisierungsmittel die bisher bekannten Testsysteme, in denen Tetrazoliumsalze als Indikatoren verwendet wurden, nicht stören, kann diese Stabilisierung für alle bekannten Teste eingesetzt werden. Beispielsweise seien der Nachweis von Milchsäure mit Lactatdehydrogenase, Alkohol mit Alkoholdehydrogenase, Glyzerin mit Glyzerindehydrogenase, Glukose mit Glukosedehyddrogenase, Acetaldehyd mit Acetaldehyddehydrogenase genannt, sowie weitere Systeme, die sich beispielsweise über die Bildung von $H_2O_2$ und dessen Reaktion mit Alkohol und Katalase zu Acetaldehyd an das vorstehende System ankoppeln lassen. Darüberhinaus können natürlich auch starke Reduktionsmittel wie beispielsweise Ascorbinsäure direkt Tetrazoliumsalze zum Formazan reduzieren.

Die erfindungsgemäßen Zubereitungen werden dem Testsystem üblicherweise als Lösung zugesetzt, für die längere Lagerung empfiehlt es sich, sie in fester Form, beispielsweise als Lyophilisate, Pulver, Tabletten oder imprägniert auf saugfähige Träger, zu verwenden. Solche saugfähigen Träger können gleichzeitig an einem kurzen Handgriff befestigt sein, so daß man sie als Mischstäbchen gemäß DE-OS 23 01 999 verwenden kann.

In den folgenden Beispielen sollen einige Anwendungsformen der erfindungsgemäßen Zubereitung

0 054 689

beschrieben sein, ohne das dadurch die Erfindung in irgendeiner Weise begrenzt ist.

## Beispiel 1

Reagenzpapier mit 3-(4,5-Dimethylthiazolyl-2-)-2,4-diphenyl-tetrazoliumbromid (MTT)

Filterpapier (Schleicher u. Schüll Nr. 598) wird mit einer Lösung folgender Zusammensetzung imprägniert und getrocknet.

| | |
|---|---|
| MTT | 0,25 Mol |
| Citronensäure | 0,50 Mol |
| Methanol ad | 1000 ml |

Es wird ein citronengelbes Material erhalten, das bei Elution in Wasser eine hellgelbe Lösung mit einem pH von 2,2 ergibt und welches vor Licht geschützt mindestens 1 Jahr bei Raumtemperatur stabil ist.

Vergleicht man in analoger Weise hergestellte Reagenzpapiere, die andere Säuren im gleichen molaren Verhältnis an Stelle von Citronensäure enthalten durch Vermessen an einem Remissions-Spektralphotometer (Zeiss DMR 21) zwischen 400—700 nm, so ist mit zunehmender Belastungsdauer eine mehr oder weniger ausgeprägte Verfärbung im Bereich 560—580 nm zu beobachten, die in nachstehender Tabelle aufgezeigt ist. Das Zersetzungsprodukt ist überwiegend Formazan.

| Säure-Variante (1 Mol Säure auf 0,5 Mol MTT) | pH nach Eluieren in Wasser | % Remission bei 578 nm | | |
|---|---|---|---|---|
| | | unbelastet | 12 Wochen bei 25° C | 36 Wochen bei 25° C |
| ohne Säure | 5,5 | 62 | 60 | 42 |
| Weinsäure | 2,1 | 95 | 80 | 52 |
| Salicylsäure | 2,2 | 67 | 64 | 55 |
| Oxalsäure | 1,5 | 85 | 78 | 40 |
| Borsäure | 4,5 | 93 | 91 | 80 |
| Citronensäure | 2,2 | 93 | 90 | 83 |

## Beispiel 2

Lactatdehydrogenase (LDH) — Farbtest mit MTT/Diaphorase

Ein Reagenzstreifen von 6 mm Breite und ca. 75 mm Länge, an welchem am unteren Ende 3 voneinander getrennte Bezirke der Fläche 6 × 6 mm befestigt sind und wovon der eine 1,3 mg MTT zusammen mit 1,3 mg Citronensäure, imprägniert auf Filterpapier (3455, Schleicher u. Schüll) aus methanolischer Lösung, der andere 4,8 mg NAD, imprägniert auf das Polyamid/Cellulose-Vlies (VS 532, Binzer) aus wäßriger Lösung und ein dritter 0,3 Einheiten Diaphorase, imprägniert auf VS 532 aus 0,05 molarem Tris-Citratpuffer, pH 7 enthält, wird in 2 ml einer 0,1-molaren Tris/HCl-Puffer-Lösung pH 8,0, die 0,5% Detergenz und 40 mmol/l Lactat enthält, eluiert.

Es entsteht eine Reagenzlösung folgender Zusammensetzung:

| | |
|---|---|
| Tris/HCl-Puffer | 0,1 mol/l |
| Detergenz | 0,5% |
| Lactat | 0,04 mol/l |
| NAD | 1,5 mmol/l |
| MTT | 1,5 mmol/l |
| Diaphorase | 130 U/l |
| Citrat | 6,0 mmol/l |

Zu 2 ml dieser Lösung wird 0,02 ml Serum zupipettiert, gut gemischt und das Gemisch 3 Minuten bei Raumtemperatur stehen gelassen.

Die LDH-Enzymreaktion wird dann durch kontinuierliche Registrierung oder Extinktionsablesungen nach bestimmten Zeitintervallen (z. B. alle 60 s) gemessen.

Ein Jahr bei Raumtemperatur unter Licht- und Feuchtigkeitsausschluß gelagerte Reagenzstreifen identische Ergebnisse. Die Analyse der Inhaltsstoffe ergab folgende Werte, bezogen auf 1 Reagenzstreifen.

| Inhaltsstoff | unbelastet | 6 Monate bei 25°C | 12 Monate bei 25°C |
|---|---|---|---|
| NAD | 4,8 mg | 4,6 mg | 4,0 mg |
| Diaphorase | 0,3 U | 0,21 U | 0,18 U |
| MTT | 1,3 mg | 1,3 mg | 1,2 mg |
| Formazan | 0,0 | 0,0 | 0,0 |

Ein bis auf den Zusatz von Citratpuffer und Citronensäure identisch hergestellter Reagenzstreifen, zeigt folgende Analysenwerte. Bereits bei unbelasteten Papieren stört das aus MTT gebildete Formazan die Messung. Nach Belastung ist eine Messung nicht mehr möglich.

| Inhaltsstoff | unbelastet | 6 Monate bei 25°C |
|---|---|---|
| NAD | 4,8 mg | 4,6 mg |
| Diaphorase | 0,3 U | 0,21 U |
| MTT | 1,2 mg | 1,0 mg |
| Formazan | 0,1 mg | 0,3 mg |

**Patentansprüche**

1. Stabilisierte Zubereitungen von Tetrazoliumsalzen für analytische Zwecke, dadurch gekennzeichnet, daß sie 1—10, vorzugsweise 1—2 Mol Borsäure oder eine organische, in polaren Lösungsmitteln lösliche Hydroxypolycarbonsäure pro Mol Tetrazoliumsalz enthalten.

2. Zubereitungen nach Anspruch 1, dadurch gekennzeichnet, daß die Säure Zitronensäure ist.

3. Zubereitungen nach Anspruch 1—2, dadurch gekennzeichnet, daß das Tetrazoliumsalz

3-(4',5'-dimethyl-thiazolyl-2-)-2,4-diphenyl-tetrazoliumbromid,
2-(p-Iodophenyl)-3-(p-nitrophenyl)-5-phenyl-tetrazoliumchlorid oder
2,2'-Di-(p-nitrophenyl)-5,5'-diphenyl-3,3'-(3,3'-dimethoxy-4,4'-diphenylen)-ditetrazoliumchlorid

ist.

4. Zubereitungen nach Anspruch 1—3, dadurch gekennzeichnet, daß die Zubereitung als Lösung, Lyophilisat, oder Imprägnierung auf einem saugfähigen Träger vorliegt.

5. Verfahren zum Nachweis von reduzierenden Stoffen, mittels Tetrazoliumsalzen und gegebenenfalls üblichen Aktivatoren wie Diaphorase oder 5-Methyl-phenazinium-methylsulfat, dadurch gekennzeichnet, daß man dem Testansatz eine stabilisierte Zubereitung gemäß Anspruch 1—5 zufügt.

6. Verfahren nach Anspruch 5, dadurch gekennzeichnet, daß man die stabilisierte Zubereitung des Tetrazoliumsalzes zu einer auf Reaktionsbedingungen gepufferten Lösung der übrigen Reagenzien zufügt und anschließend die Probe zusetzt und die Farbveränderung mißt.

7. Verwendung von Zubereitungen nach Anspruch 1—4, zum Nachweis von NADH,NADH-bildenden Systemen oder Ascorbinsäure.

## Claims

1. Stabilised compositions of tetrazolium salts for analytical purposes, characterised in that they contain 1—10, preferably 1—2 mole of boric acid or an organic hydroxypolycarboxylic acid soluble in polar solvents per mole of tetrazolium salt.

2. Compositions according to claim 1, characterised in that the acid is citric acid.

3. Compositions according to claim 1—2, characterised in that the tetrazolium salt is
   3-(4',5'-dimethylthiazolyl-2)-2,4-diphenyltetrazolium bromide,
   2-(p-iodophenyl)-3-(p-nitrophenyl)-5-phenyltetrazolium chloride or
   2,2'-di-(p-nitrophenyl)-5,5'-diphenyl-3,3'-(3,3'-dimethoxy-4,4'-diphenylene)-ditetrazolium chloride.

4. Compositions according to claim 1—3, characterised in that the composition is present as solution, lyophilisate or impregnation on an absorbent carrier.

5. Process for the detection of reducing materials by means of tetrazolium salts and optionally conventional activators, such as diaphorase or 5-methyl-phenazinium-methylsulphate, characterised in that one adds to the test batch a stabilised composition according to claim 1—5.

6. Process according to claim 5, characterised in that one adds the stabilised composition of the tetrazolium salt to a solution, buffered to reaction conditions, of the other reagents and subsequently adds the sample and measures the colour change.

7. Use of compositions according to claim 1—4 for the detection of NADH,NADH-forming systems or ascorbic acid.

## Revendications

1. Préparations stabilisées de sels de tétrazolium à des fins analytiques, caractérisées en ce qu'elles contiennent entre 1 et 10 moles, de préférence entre 1 et 2 moles d'acide borique ou d'un acide hydroxypolycarboxylique organique soluble dans des solvants polaires, par mole de sel de tétrazolium.

2. Préparations selon la revendication 1, caractérisées en ce que l'acide est l'acide citrique.

3. Préparations selon la revendication 1 ou 2, caractérisées en ce que le sel de tétrazolium est le bromure de

   3-(4',5'-diméthyl-thiazolyl-2-)-2,4-diphényl-tétrazolium,
   le chlorure de 2-(p-iodophényl)-3-(p-nitrophényl)-5-phényltétrazolium ou le chlorure de
   2,2'-di(p-nitrophényl)-5,5'-diphényl-3,3'-(3,3'-diméthoxy-4,4'-diphénylène)ditétrazolium.

4. Préparations selon l'une quelconque des revendications 1 à 3, caractérisées en ce que la préparation se présente sous forme d'une solution, d'un lyophilisat ou d'un support absorbant imprégné.

5. Procédé pour la mise en évidence de matières réductrices au moyen de sels de tétrazolium et éventuellement des acitvateurs habituels comme la diaphorase ou le méthylsulate de 5-méthyl-phénazinium, caractérisées en ce qu'on ajoute au produit à tester une préparation stabilisée selon l'une quelconque des revendications 1 à 4.

6. Procédé selon la revendication 5, caractérisé en ce qu'on ajoute la préparation stabilisée du sel de tétrazolium à une solution des autres réactifs portée au moyen d'un tampon aux conditions réactionnelles et en ce qu'on ajoute ensuite l'échantillon à examiner et on mesure le changement de la couleur.

7. Utilisation des préparations selon l'une quelconque des revendications 1 à 4 pour la mise en évidence du NADH, de systèmes formant du NADH ou de l'acide ascorbique.